# FASCICULE DE BREVET EUROPEEN

(11) **EP 3 729 455 B1**
(45) Date de publication et mention de la délivrance du brevet: **29.11.2023**
(21) Numéro de dépôt: 18839695.6
(22) Date de dépôt: 17.12.2018
(51) Int. Cl.: G16H 40/63, G09B 23/30, G16H 20/40

(54) **PROCÉDÉ DE DÉTERMINATION DU POSITIONNEMENT EN POSITION DÉPLOYÉE D'UN DISPOSITIF MÉDICAL IMPLANTABLE APRÈS EXPANSION DANS UNE STRUCTURE VASCULAIRE**
VERFAHREN ZUR BESTIMMUNG DER ENTFALTETEN POSITION EINES IMPLANTIERBAREN MEDIZINPRODUKTS NACH DER EXPANSION IN EINER GEFÄSSSTRUKTUR
METHOD FOR DETERMINING THE DEPLOYED POSITION OF AN IMPLANTABLE MEDICAL DEVICE AFTER EXPANSION IN A VASCULAR STRUCTURE

(30) Priorité: 19.12.2017 FR 1762490
(43) Date de publication de la demande: 28.10.2020
(73) Titulaire: Sim&Cure, 34090 Montpellier (FR)
(72) Inventeur: FERRARA, Riccardo, 34090 Montpellier (FR); SANCHEZ, Mathieu, 34660 Cournonterral (FR); COSTALAT, Vincent, 34980 Saint-Gély-du-Fesc (FR); AMBARD, Dominique, 34150 Aniane (FR); CHNAFA, Christophe, 34090 Montpellier (FR); SIGUENZA, Julien, 34090 Montpellier (FR)
(74) Mandataire: Regimbeau
(86) Numéro de dépôt international: PCT/FR2018/053343
(87) Numéro de publication internationale: WO 2019/122665

(56) Documents cités:
- EP-A1- 3 025 638
- DE-A1-102006 058 908
- US-A1- 2003 195 411
- JIN DAKAI ET AL: "A New Approach of Arc Skeletonization for Tree-like Objects Using Minimum Cost Path", INTERNATIONAL CONFERENCE ON PATTERN RECOGNITION, IEEE COMPUTER SOCIETY, US, 24 août 2014 (2014-08-24), pages 942-947, XP032698628, ISSN: 1051-4651, DOI: 10.1109/ICPR.2014.172 [extrait le 2014-12-04]
- Ding Ma ET AL: "High Fidelity Virtual Stenting (HiFiVS) for Intracranial Aneurysm Flow Diversion: In Vitro and In Silico", Annals of Biomedical Engineering, vol. 41, no. 10, 20 April 2013 (2013-04-20), pages 2143-2156, XP055275788, New York ISSN: 0090-6964, DOI: 10.1007/s10439-013-0808-4
- Ding Ma ET AL: "Computer modeling of deployment and mechanical expansion of neurovascular flow diverter in patient-specific intracranial aneurysms", JOURNAL OF BIOMECHANICS, vol. 45, no. 13, 1 August 2012 (2012-08-01), pages 2256-2263, XP055275656, US ISSN: 0021-9290, DOI: 10.1016/j.jbiomech.2012.06.013
- Anonymous: "vmtk - the Vascular Modeling Toolkit | Tutorials / Centerlines", , 8 May 2009 (2009-05-08), XP55858605, Retrieved from the Internet: URL:https://web.archive.org/web/2009050816 5358/http://www.vmtk.org/Tutorials/Centerl ines [retrieved on 2021-11-08]
- Di Dipartimento ET AL: "POLITECNICO DI MILANO Patient-Specific Modeling of Geometry and Blood Flow in Large Arteries Co-Advisers", , 31 December 2002 (2002-12-31), XP55858607, Retrieved from the Internet: URL:https://lantiga.github.io/media/Antiga PhDThesis.pdf [retrieved on 2021-11-08]
- Anonymous: "Computing Centerlines | vmtk - the Vascular Modelling Toolkit", , 19 March 2014 (2014-03-19), XP055927501, Retrieved from the Internet: URL:https://web.archive.org/web/2014031910 1222/http://www.vmtk.org/tutorials/Centerl ines.html [retrieved on 2022-06-02]

## Description

### DOMAINE TECHNIQUE DE L'INVENTION ET ETAT DE LA TECHNIQUE

La présente invention s'inscrit dans le domaine de la simulation numérique d'implants vasculaires, avant ou pendant l'opération de pose.

Plus particulièrement, l'invention concerne la prédiction du comportement mécanique d'un dispositif médical implantable (ou DMI), pour lequel on dispose d'une cartographie tridimensionnelle d'une structure vasculaire, telle qu'une artère, subissant une pathologie locale comme un anévrisme, avant la pose dudit implant par voie endovasculaire. Cette prédiction est utile au clinicien pour le choix d'une taille optimale et d'un positionnement initial optimal du dispositif, voire le choix d'un modèle optimal, lors de l'intervention.

Il est courant d'utiliser un implant de type dispositif médical implantable (DMI) expansible, tel qu'un « stent », une « cage intra-sacculaire », ou un « flow diverter » selon la terminologie anglosaxonne couramment utilisée, pour traiter par exemple une artère touchée par un anévrisme. On souhaite éviter l'expansion et la rupture de l'anévrisme, et par ailleurs, éviter que des caillots formés dans le sac anévrismal ne migrent et ne bouchent localement une artère.

Un tel implant peut être posé par implantation percutanée. L'implant peut être conservé à un état radialement compressé dans un cathéter, et se déployer après positionnement dans une zone à traiter. Le DMI peut être de forme cylindrique, sphérique, ellipsoïde ou de forme tubulaire à rayon variable autour de son axe de révolution.

Il est important de prévoir, avant la pose, la position déployée adoptée par l'implant. Par exemple pour le cas d'un « flow diverter », l'implant peut s'étendre (par rapport à sa longueur au repos) le long de son axe longitudinal après la pose de l'implant. Pour le cas d'une cage intra-sacculaire ou intra-anévrismale à géométrie variable, l'implant peut s'étendre longitudinalement et radialement autour de son axe de révolution après le déploiement de celui-ci dans le sac anévrismal, de sorte que l'implant après expansion épouse la forme du sac anévrismal jusqu'à l'équilibre mécanique. Pour le traitement d'un anévrisme, la longueur finale de l'implant, son positionnement exact, son diamètre, son apposition contre les parois de l'artère sont autant de paramètres dont la maîtrise est importante. Un dispositif se déployant sur une longueur trop importante peut couvrir une bifurcation artérielle de façon non désirée. Un dispositif de longueur trop faible peut traiter de manière insuffisante la pathologie anévrismale. De plus, si l'apposition du dispositif contre les parois de l'artère est insatisfaisante, il existe un risque de formation d'un thrombus.

La planification d'une pose d'implant par voie endovasculaire est généralement réalisée à l'aide d'images bidimensionnelles ou tridimensionnelles de l'artère. Jusqu'à présent, le choix de la référence de DMI à déployer est basé - à minima - sur des mesures locales planaires (2D) effectuées par le médecin sur l'imagerie du patient. Ces mesures ne permettent pas de prédire une apposition du DMI ou sa longueur finale une fois déployé de manière fiable.

Afin de prendre en compte la morphologie de la structure vasculaire pour estimer la position finale d'un implant endovasculaire après déploiement, on connaît des techniques fondées sur l'utilisation d'abaques. Les abaques donnent une relation entre la longueur du dispositif après déploiement et son diamètre. En supposant que l'expansion de l'implant soit limitée par le diamètre minimal de la structure vasculaire qui l'entoure, il est possible d'obtenir une estimation de longueur après déploiement. Cependant, cette technique présuppose une structure vasculaire ayant une section circulaire constante.

Il existe donc un besoin pour un procédé de détermination de la position finale d'un implant après déploiement, et notamment de son expansion radiale après déploiement, qui soit très précis et fiable. Il existe un besoin additionnel pour un modèle capable de donner une estimation de l'apposition de l'implant contre les parois de la structure vasculaire.

"High Fidelity Virtual Stenting (HiFiVS) for Intracranial Aneurysm Flow Diversion: In Vitro and In Silico" de Ding Ma et Al. et EP 3 025 638 A1 décrivent des procédés de détermination du positionnement en position déployée d'un dispositif médical implantable expansible, ledit dispositif médical étant destiné à être déployé dans une structure vasculaire, formant part de l'état de l'art.

### PRESENTATION GENERALE DE L'INVENTION

L'invention permet de pallier les inconvénients pré-cités et propose, selon un premier aspect, un procédé de détermination du positionnement en position déployée d'un dispositif médical implantable, ou DMI, expansible, ledit dispositif médical étant destiné à être déployé dans une structure vasculaire, le procédé comprenant les étapes de la revendication 1.

Les avantages de l'invention sont multiples.

L'invention, qui s'applique pour la simulation d'un implant chez l'homme comme chez l'animal, améliore la détermination de la ligne centrale, une image tridimensionnelle de la structure vasculaire étant utilisée pour déterminer les points de la ligne centrale. De plus, l'utilisation d'un algorithme de descente de gradient permet d'obtenir une ligne centrale plus proche de la réalité physique. La ligne centrale ainsi déterminée est plus fidèle à l'axe longitudinal réel de l'implant après déploiement.

Cette ligne centrale sert de base pour la correction de la position du DMI, pour déterminer une position finale du DMI, et notamment sa longueur après déploiement.

La prise en compte d'un taux de compression longitudinale, lors de la correction de la position du DMI, rend cette correction plus fiable puisqu'on tient compte de la contrainte mécanique exercée lors de la pose de l'implant, pouvant aller par exemple dans le sens d'une expansion radiale de l'implant. On modélise alors les cas où l'implant adopte localement une position d'équilibre différente d'une forme de cylindre de révolution. En effet, pour un taux de compression longitudinale non nul, la force radiale exercée par l'implant rapproche les parois de l'implant et la surface de la structure vasculaire .

Des caractéristiques additionnelles et non-limitatives d'un procédé selon le premier aspect de l'invention sont celles définies dans chacune des revendications 2 à 12, prises seules ou en l'une quelconque de leurs combinaisons techniquement possibles.

Selon un deuxième aspect, l'invention concerne un produit programme d'ordinateur comprenant des instructions de code pour l'exécution d'un procédé tel que décrit ci-avant, lorsqu'il est exécuté par des moyens de traitement d'une unité de traitement.

Selon un autre aspect, l'invention vise une unité de traitement configurée pour obtenir une image tridimensionnelle de structure vasculaire acquise d'une unité d'acquisition, et configurée en outre pour simuler une position finale d'un DMI expansible, par la mise en oeuvre d'un procédé tel que décrit ci-avant.

### PRESENTATION GENERALE DES FIGURES

D'autres caractéristiques, buts et avantages de l'invention ressortiront de la description qui suit, qui est purement illustrative et non limitative, accompagnée des dessins annexés parmi lesquels :
La **Figure 1** illustre un système propre à exécuter un procédé selon l'invention ;
La **Figure 2** illustre une séquence d'étapes d'un procédé de détermination de position finale d'implant selon l'invention ;
La **Figure 3a** est une angiographie d'artère atteinte d'un anévrisme ;
La **Figure 3b** correspond à une vue tridimensionnelle de la même artère, dans laquelle on a représenté la ligne centrale de l'artère ;
La **Figure 3c** correspond à la même vue tridimensionnelle dans laquelle on a représenté un DMI de type « flow diverter » ;
La **Figure 4a** illustre une séquence d'étapes d'un procédé selon un mode de réalisation, pour la détermination de la position d'un « flow diverter » après déploiement ;
La **Figure 4b** illustre une séquence d'étapes d'une correction de déploiement géométrique, pour la détermination de la position d'un DMI après déploiement ;
La **Figure 4c** illustre une séquence d'étapes d'une correction de déploiement mécanique, pour la détermination de la position d'un DMI après déploiement ;
Les **Figures 5a, 5b****,** **5c et 5d** illustrent schématiquement des itérations successives au sein d'une étape de simulation de position finale d'un « flow diverter » tenant compte de contraintes géométriques ;
La **Figure 6** illustre schématiquement des sections transversales d'une artère équipée d'un DMI de type « flow diverter », dans plusieurs cas possibles ;
La **Figure 7** illustre une cartographie tridimensionnelle du degré d'apposition prévu pour un DMI.
La **Figure 8a** illustre le résultat de l'étape de positionnement du DMI dans sa position initiale, la **Figure 8b** illustre le résultat de l'étape de simulation géométrique de position finale du DMI et la **Figure** 8c illustre le résultat de l'étape de simulation d'une correction mécanique.

### DESCRIPTION DETAILLEE DE MODES DE REALISATION

La description ci-après concerne la détermination de la position finale d'un DMI au sein d'une artère. Toutefois, l'invention peut être utilisée avec les mêmes avantages pour toute structure vasculaire du corps humain ou animal.

Dans toute la suite, on se référera indifféremment à un « implant » ou un « dispositif médical implantable » (DMI) comme étant un implant expansible, pouvant adopter une position finale au sein d'une artère qui est différente de sa position initiale (avant déploiement), et qui est également différente de sa position au repos (déploiement à l'air libre) ou « position nominale ». On entend par « caractéristiques morphologiques de l'artère » un ensemble de caractéristiques influant localement sur la position finale de l'implant (notamment mais non limitativement diamètre minimal, périmètre, rayons de courbure et leurs dérivées spatiales). Par ailleurs une région d'intérêt, comprenant l'artère à traiter, pourra être désignée « ROI ».

Par ailleurs, la description qui suit concerne spécifiquement une prothèse de type « flow diverter », de forme sensiblement cylindrique ou tubulaire. Cet implant est formé d'un maillage en matériau biocompatible pour les tissus humains. Il est maintenu dans une position radialement comprimée et longitudinalement allongée, par exemple dans un cathéter, avant la pose. Toutefois, un procédé selon l'invention peut être appliqué avec les mêmes avantages pour tout type d'implant expansible autre que le « flow diverter », par exemple pour une cage intra-anévrismale (dispositif intra-sacculaire), pour un dispositif de forme conique, ou encore pour un dispositif de type stent, par exemple un « stent laser-cut » selon la terminologie anglosaxonne courante.

### Système de détermination du positionnement en position déployée d'un DMI

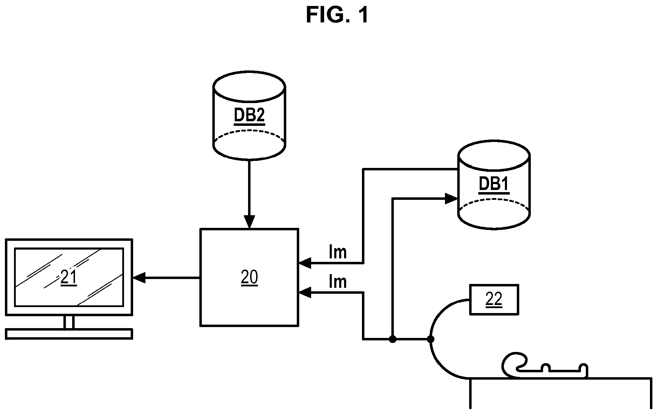

On a représenté en **Figure 1** un système de détermination du positionnement d'un DMI comprenant une unité de traitement 20 selon l'invention. Cette unité de traitement est, par exemple, un processeur configuré pour mettre en oeuvre un procédé de détermination du positionnement d'un DMI qui sera décrit ci-après.

Les moyens de traitement sont, de manière avantageuse, configurés pour communiquer avec une unité d'acquisition 22, configurée pour acquérir des vues permettant de reconstituer une image tridimensionnelle d'une région d'intérêt d'un patient, ladite région d'intérêt comprenant une artère, et/ou pour communiquer avec une base de données DB1 dans laquelle sont enregistrées des images tridimensionnelles acquises sur un patient et/ou des vues permettant de reconstituer de telles images tridimensionnelles. L'unité d'acquisition 22 peut par exemple être un système d'imagerie à rayons X, et les vues peuvent par exemple être acquises dans le cadre d'une procédure de neuro-radiologie comme par exemple l'acquisition d'une angiographie 3D. L'unité de traitement 20 communique avec l'unité d'acquisition 22 et/ou avec la base de données DB1 de sorte à recevoir des images Im, par liaison filaire et/ou par liaison sans fil à l'aide de tout réseau adapté (réseau Internet, etc). En alternative, l'unité de traitement peut extraire les images Im d'un disque dur, ou recevoir les images Im par l'intermédiaire d'un périphérique de lecture de support de stockage tel qu'un lecteur de CD ou un port USB.

Les moyens de traitement sont en outre configurés pour communiquer avec une base de données DB2 comprenant des données concernant des dispositifs médicaux implantables, ou DMI. Ces données peuvent par exemple être des caractéristiques physiques de DMI, ou des modèles préenregistrés de DMI. Lesdites données peuvent être fournies par des fabricants de DMI, ou déterminées analytiquement ou expérimentalement. La liaison entre l'unité de traitement 20 et la base de données DB2 est réalisée par tout moyen adapté.

Enfin, l'unité de traitement 20 peut communiquer avec un dispositif d'affichage 21, fournissant une interface graphique pour affichage d'images tridimensionnelles de régions d'intérêt et pour tout autre affichage nécessaire à la mise en oeuvre du procédé qui sera décrit ci-après. Le dispositif d'affichage 21 est associé à une interface utilisateur pour la saisie d'instructions et de données.

### Procédé de détermination du positionnement en position déployée d'un DMI

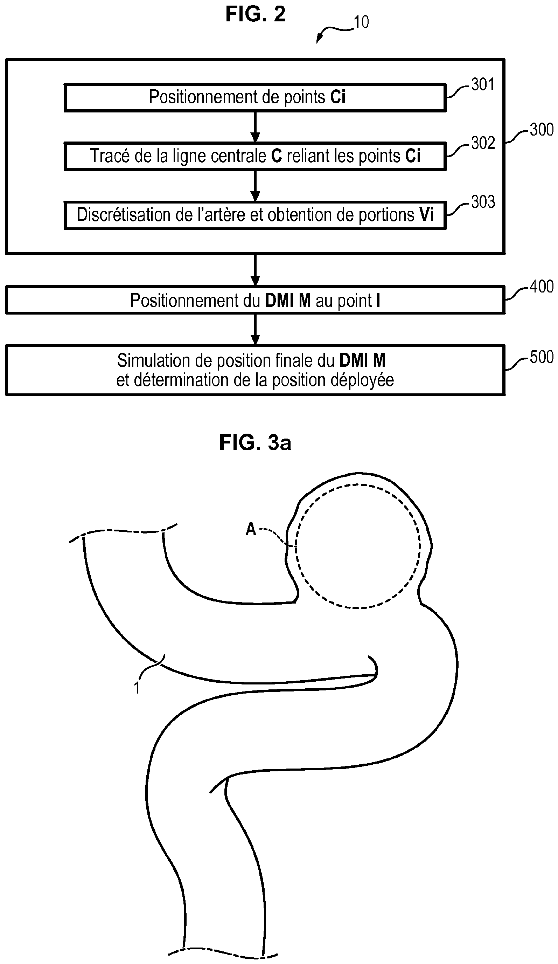

On a représenté en **Figure 2** des étapes d'un procédé 10, constituant un exemple de mise en oeuvre d'un procédé de l'invention pour la détermination de la position déployée d'un implant extensible au sein d'une artère.

Dans ce qui suit, le conduit corporel traité par la pose d'un implant est une artère, et l'implant est un implant endovasculaire ; toutefois, l'invention pourrait être utilisée avec les mêmes avantages pour une prothèse destinée au traitement de tout autre conduit corporel, chez l'humain ou l'animal. On a représenté en **Figure 3a** une vue bidimensionnelle correspondant à une angiographie d'une artère 1 à traiter, présentant en une zone A un sac anévrismal.

Cette image est obtenue, par exemple par une unité de traitement reliée à un scanner à rayons X ou par tout moyen connu du même type.

Préalablement aux étapes du procédé 10, on détermine un point de positionnement initial D de l'implant, appelé par exemple « point distal » et, optionnellement, un deuxième point de positionnement final P appelé par exemple « point proximal ». Ces deux points peuvent, dans le cas d'un flow diverter, correspondre aux positions des deux extrémités souhaitées par le clinicien. Une référence de DMI est sélectionnée, de manière automatique ou manuellement par un opérateur, ladite référence correspondant à un type d'implant avec des dimensions préenregistrées ou sélectionnées manuellement. Le procédé 10 peut également prévoir une sélection automatique d'une référence d'implant adaptée avec des dimensions adaptées, en fonction d'informations extraites de l'image tridimensionnelle de l'artère, et/ou en fonction d'une distance entre le point proximal et le point distal. Dans la suite, on considère que la référence d'implant sélectionnée correspond à un « flow diverter ». Le procédé 10 prend par ailleurs en entrée une image tridimensionnelle Im de l'artère. Nous ne détaillons pas ici le protocole pour obtenir ladite image tridimensionnelle d'artère.

Le procédé 10 comprend une étape 300 de détermination d'une ligne centrale C de l'artère, au moins au niveau de la zone A. En effet, pour avoir accès à la position déployée de l'implant, il convient de déterminer l'axe le long duquel l'implant peut s'étendre ou se rétracter au sein de l'artère. Cet axe correspond globalement à la ligne centrale (en anglais, « centerline ») de l'artère. La ligne centrale C est déterminée à l'étape 300 en plaçant des points Ci à des positions longitudinales différentes le long de l'artère, au sein du volume interne de l'image tridimensionnelle Im de l'artère.

Les points Ci sont placés le long de l'artère de sorte à obtenir un chemin le plus rapide entre un point d'entrée I dans l'artère, typiquement un point d'entrée d'un micro-cathéter pour réaliser l'implantation du DMI au niveau de la région d'intérêt, et un point de sortie de l'artère. Les points d'entrée et de sortie peuvent être entrés manuellement par un opérateur par l'intermédiaire de l'interface du dispositif 21, ou peuvent être déterminés automatiquement sur l'image tridimensionnelle de l'artère.

Par « chemin le plus rapide » on entend une minimisation du temps de trajet pour le sang entre le point I et le point de sortie.

De façon très avantageuse, le placement des points Ci à partir du point I est réalisé en appliquant un algorithme de descente de gradient, pour minimiser le temps de trajet le long des points ainsi placés.

A partir d'un point courant parmi les points Ci, on recherche le point suivant de la ligne centrale dans la direction opposée au gradient de la fonction de temps de trajet.

La fonction de temps de trajet correspond, par exemple, à une distance le long d'un arc de courbe tridimensionnelle correspondant à la ligne centrale, multipliée par une fonction de coût ayant la dimension de l'inverse d'une vitesse.

Selon un mode de réalisation particulier représenté en Figure 2, l'étape 300 comprend une sous-étape 301 pendant laquelle l'unité de traitement calcule successivement des points Ci le long de l'artère. Si l'artère était parfaitement cylindrique, les points Ci seraient placés successivement le long de l'axe de révolution du cylindre formant alors l'artère. Dans la réalité, l'artère n'est pas localement cylindrique. Le positionnement des points Ci est donc réalisé en cherchant à minimiser la distance entre chaque point Ci et la surface avoisinanteᵢ de l'artère. En outre, la détermination d'un point Ci de ligne centrale est aussi réalisée en cherchant à minimiser le temps de trajet de fluide le long des points de ligne centrale déjà déterminés. Les points Cᵢ peuvent être obtenus sur des portions Vᵢ consécutives considérées dans l'ordre.

A une sous-étape 302, la ligne centrale C reliant les points Ci est tracée sur l'image tridimensionnelle de l'artère. L'étape 302 peut comprendre un calcul de trajet optimal entre des points Ci déterminés précédemment, et la ligne centrale correspond alors audit trajet optimal. Le trajet optimal peut être par exemple le trajet le plus rapide dans l'artère entre l'entrée du micro-cathéter au sein de l'artère (point I) et le point de sortie de l'artère (point S).

Enfin, à une étape 303, l'unité de traitement réalise l'extraction, à partir de l'image tridimensionnelle de l'artère, de portions tridimensionnelles Vᵢ de l'artère, le long de la ligne centrale précédemment déterminée. Avantageusement, les portions extraites le long de la ligne centrale correspondent à des tronçons d'artère, pouvant avoir une forme tubulaire à section circulaire.

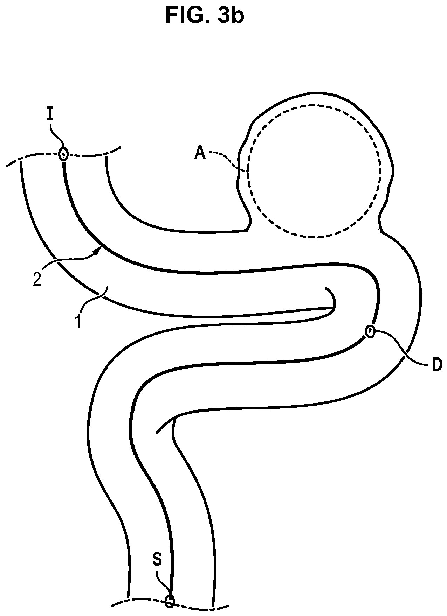

La **Figure 3b** est une vue d'un modèle tridimensionnel pour l'artère 1 de la Figure 3a. Sur la vue de la Figure 3b, on a représenté la ligne centrale 2 obtenue pour l'artère 1 à l'issue de l'étape 300. On a en outre repéré le point initial D de positionnement d'un DMI. La vue de la Figure 3b correspond à une représentation tridimensionnelle de l'artère qui peut être fournie sur le dispositif d'affichage 21 lors de l'exécution du procédé 10.

Le procédé 10 comprend ensuite une étape 400 de placement d'un DMI M, correspondant à la référence d'implant prise en entrée du procédé. L'extrémité distale du DMI M est placée au point initial D (point distal), qui est avantageusement visible sur l'image tridimensionnelle. Par exemple, le point distal D peut être positionné en périphérie de la zone A à traiter. Le DMI M est placé autour de la ligne centrale C déterminée à l'étape 300. Par « autour de » on entend que les parois du DMI M entourent la ligne centrale, même si le DMI n'est pas nécessairement centré sur la ligne centrale. Si le DMI M correspond à un « flow diverter », le DMI M peut être pris de forme tubulaire avec son axe longitudinal coïncidant avec la ligne centrale de l'artère. Le point D constitue l'extrémité distale du DMI M. Les dimensions du DMI M placé au point D correspondent aux dimensions de l'implant au repos, qui ne sont pas nécessairement identiques aux dimensions après déploiement qui seront déterminées à la suite du procédé.

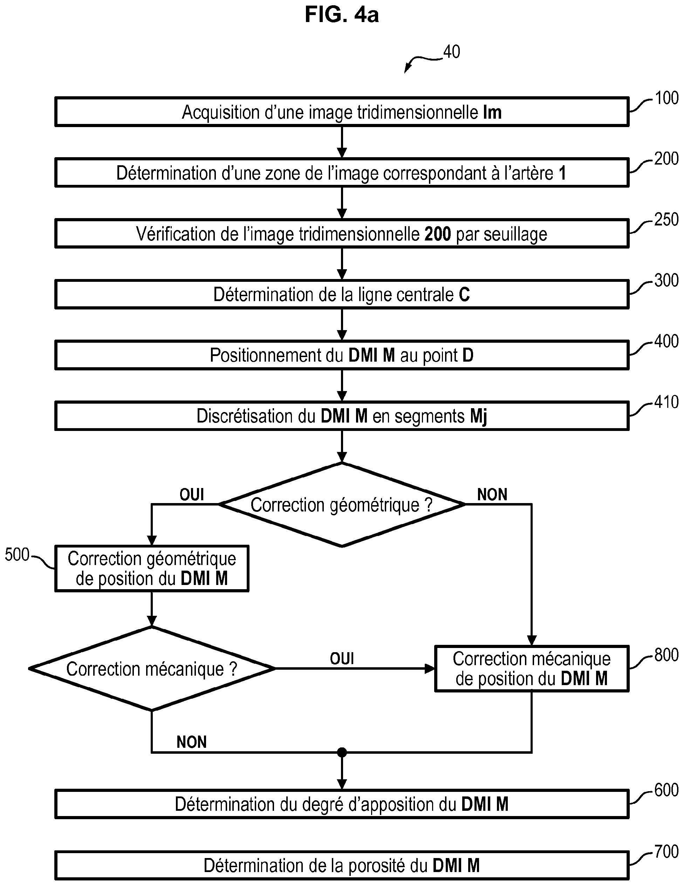

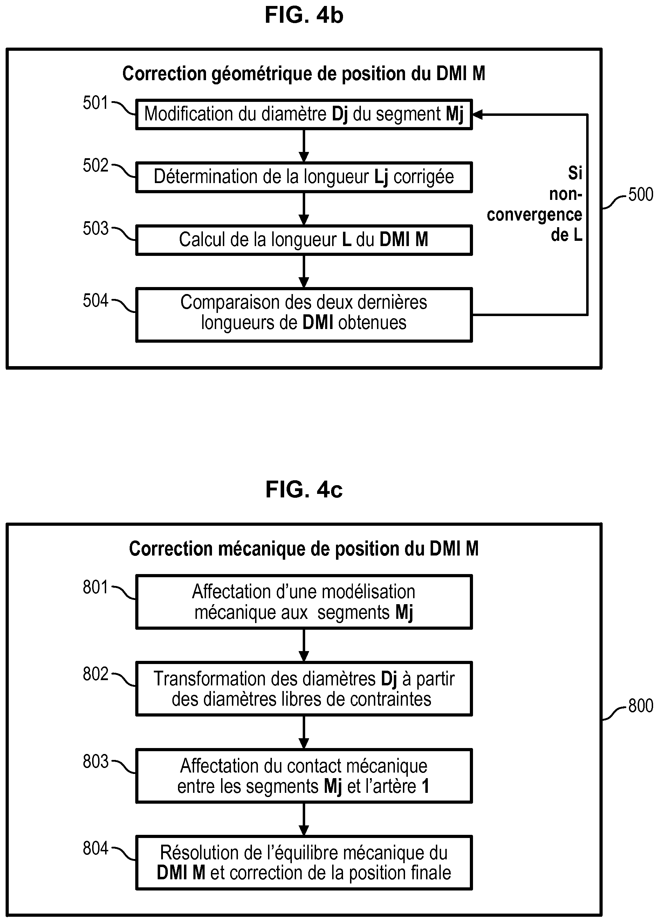

Ces dimensions, propres à chaque référence de DMI M, peuvent être sélectionnées automatiquement en fonction des positions du point D et du point proximal, de la distance entre la ligne centrale déterminée à l'étape 300 et les parois de l'artère, ou en fonction de caractéristiques morphologiques de l'artère, ou encore manuellement. La référence du DMI M peut également être modifiée en fonction d'une première estimation de l'apposition des parois du modèle M de DMI contre les parois de l'artère. La position initiale du DMI M à l'issue de l'étape 400 correspond à une position hors contrainte, où le DMI n'est pas sollicité dans le sens d'une compression radiale ou d'une compression longitudinale par les parois environnantes de l'artère. A l'étape 500, la position du DMI M est corrigée en fonction des caractéristiques morphologiques de l'artère, de manière à tenir compte des contraintes mécaniques exercées par les parois de l'artère pour obtenir une position finale après déploiement. Avantageusement, localement, la longueur et/ou le diamètre du DMI M sont corrigés. Avantageusement, le DMI M peut être discrétisé en un ensemble de segments connexes afin de réaliser l'opération de simulation de la position finale du DMI M. Un tel cas sera détaillé ci-après en relation au procédé 40 de la figure 4.

Après simulation de la position finale du DMI M, la position obtenue correspond à la position déterminée du DMI après déploiement. Son extension peut être spontanée, ou bien être provoquée lors d'une opération de pose de l'implant. La position finale simulée doit donc permettre à un opérateur de constater si la référence d'implant choisie et la taille initiale choisie sont adaptées pour le traitement de l'artère. Il peut s'agir notamment d'observer si la pathologie vasculaire visée est correctement traitée, et si l'implant après expansion ne produirait pas des effets secondaires indésirables sur d'autres zones d'un arbre vasculaire visible sur l'image tridimensionnelle.

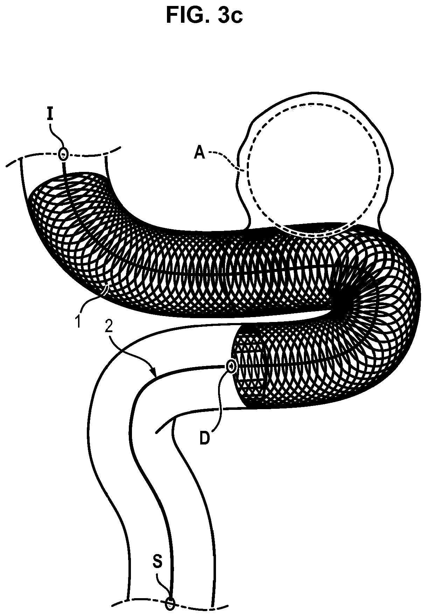

La **Figure 3c** reprend la représentation tridimensionnelle de la Figure 3b, dans laquelle on a représenté le DMI M d'implant après la mise en oeuvre de l'étape 500 pour une référence d'implant de type « flow diverter ». La Figure 3c correspond à une vue fournie sur une interface graphique reliée à l'unité de traitement. On discerne sur la vue de la Figure 3c trois zones d'implantation du « flow diverter » : une première zone où l'implant adopte une forme globalement tubulaire de rayon légèrement variable et une ligne centrale 2 légèrement déviée, une deuxième zone où l'implant adopte une position coudée, et une troisième zone où l'implant adopte à nouveau une forme globalement tubulaire. L'apposition de l'implant est plus faible au niveau du collet de l'anévrisme sacculaire ici représenté. En revanche, l'implant est correctement apposé sur la paroi d'artère opposée à l'anévrisme. Bien qu'il ne soit pas fourni sur la vue de la Figure 3c une représentation d'un degré d'apposition de l'implant, une telle représentation pourrait être fournie ici, par exemple selon les modalités qui seront décrites ci-après en relation au procédé 40.

On a représenté en **Figure 4a** des étapes d'un procédé 40 de détermination de position d'un implant de type « flow diverter » après expansion au sein d'une artère, selon un mode de réalisation particulier du procédé de la Figure 2. Des références numériques identiques entre les figures correspondent à des étapes similaires ou à des éléments similaires. Ce procédé peut également être mis en oeuvre par l'unité de traitement 20 représentée en **Figure 1****.**

Dans une étape 100, une vue tridimensionnelle Im d'une artère à traiter est acquise, par exemple par angiographie. L'image tridimensionnelle de l'artère peut être segmentée par la méthodologie des « marching cubes » connue dans le domaine de la reconstruction d'image. On pourra se référer pour la construction d'image tridimensionnelle, à la publication suivante : Marching Cubes : A high resolution 3D surface construction algorithm, William E. Lorensen, Harvey E. Cline, Computer Graphics, 1987.

A une étape 200, une zone A est repérée, manuellement par un opérateur ou automatiquement, sur la vue tridimensionnelle obtenue à l'issue de l'étape 100. Cette zone A correspond à une artère à traiter par la pose d'un implant, et peut notamment correspondre à une pathologie anévrismale de type sacculaire ou fusiforme, ou tout autre type de pathologie anévrismale, ou un rétrécissement localisé de l'artère, ou une sténose, ou une thrombose.

A une étape optionnelle 250, l'image tridimensionelle Im est vérifiée. On peut notamment vérifier la qualité de l'image Im par seuillage : l'image tridimensionnelle Im est superposée à une radiographie bidimensionnelle vue sous le même angle, à laquelle on applique un seuillage. L'image tridimensionnelle peut être corrigée en fonction du résultat de la comparaison à l'image bidimensionnelle seuillée, notamment pour vérifier la dimension de l'arbre artériel. L'image tridimensionnelle Im peut également être remise à l'échelle par rapport à la radiographie bidimensionnelle.

Il est à noter que les étapes 100 à 250 sont optionnelles pour l'exécution du procédé 40. Le procédé 40 pourrait être exécuté par une unité de traitement recevant en entrée une image tridimensionnelle de l'artère à traiter avec une zone A.

A une étape 300, une ligne centrale C ou « centreline » de l'artère est déterminée par les moyens de traitement. La ligne centrale ainsi obtenue traverse la zone à traiter. Très avantageusement, la ligne centrale peut être déterminée selon les modalités décrites précédemment en relation au procédé 10 de la Figure 2.

A une étape optionnelle (non représentée sur la figure 2), à partir de l'image tridimensionnelle de l'artère et de la ligne centrale déterminée à l'étape 300, l'unité de traitement peut, si nécessaire, réaliser une correction d'un abouchement non présent sur l'artère réelle à traiter, comme une fusion artère-artère ou une fusion artère-sac anévrismal. Une telle erreur peut se produire lors de la reconstruction et peut également être corrigée par l'opérateur.

A une étape 400, un DMI M correspondant à une référence présélectionnée de « flow diverter » avec des dimensions au repos est positionné en un point initial D, de manière semblable à l'étape 400 du procédé 10. Le DMI M est avantageusement pris de forme tubulaire avec une section de rayon constant, le long de la ligne centrale C déterminée à l'étape 300 et centré sur ladite ligne centrale. Le DMI M selon ce positionnement initial peut déborder des parois de l'artère en certaines zones.

A une étape 410, le DMI M est discrétisé en une pluralité de segments Mⱼ, où l'indice j indexe les segments consécutifs du DMI M. De manière très avantageuse, chacun des segments Mⱼ du modèle discrétisé est approximé par les moyens de traitement par un volume de forme cylindrique. Si le DMI M obtenu à l'issue de l'étape 400 est tubulaire à section de rayon constant, tous les segments Mⱼ correspondent à des tubes de même rayon centrés sur la ligne centrale C. Le nombre de segments peut être prédéterminé et indépendant des caractéristiques morphologiques de l'artère, et peut par exemple être fixé à 200. La longueur de tous les segments peut être la même et être prise égale à la longueur de la ligne centrale divisée par le nombre de segments. En alternative, la longueur de chacun des segments peut ne pas être choisie uniformément. Par exemple, la longueur des segments peut être choisie de façon à obtenir un maillage plus fin sur des zones où le périmètre des parois de l'artère est très variable, ou bien sur des zones où la ligne centrale est très déviée. La discrétisation du DMI M peut être réalisée depuis le point initial D, et donc depuis une extrémité de la position initiale du DMI M, jusqu'à l'autre extrémité.

Le modèle discrétisé en segments obtenu à l'issue de l'étape 410 est utilisé pour réaliser une simulation de la position finale du DMI M. Cette simulation se fait en tenant compte de contraintes soit géométriques, soit mécaniques, soit géométriques et mécaniques, exercées par les parois de l'artère sur le DMI M. Un but de la prise en compte de ces contraintes est d'obtenir une position finale simulée correspondant à une position de l'implant après son expansion au sein de l'artère. Considérant le temps de simulation d'une correction mécanique dans le cas général, le fait de tenir compte uniquement dans un premier temps de contraintes géométriques exercées par les parois (sans chercher un équilibre mécanique au niveau des parois du DMI) permet d'économiser du temps de calcul.

Nous allons ici détailler, en relation à la **Figure 4b****,** l'étape de simulation de position finale selon un mode de réalisation 500, dans un premier cas où l'on tient compte de contraintes géométriques.

A l'étape 500, chaque segment Mⱼ est corrigé en longueur et en diamètre, en tenant compte de la variation du rayon intra-artériel. Comme mentionné ci-avant, chaque segment peut être approximé par un volume tubulaire. Pour chacun de ces segments, on applique une loi comportementale du DMI reliant mécaniquement la longueur et le diamètre, ladite loi relative par exemple à la référence d'implant préalablement sélectionnée. Cette loi de comportement peut être linéaire ou affine ou non linéaire, et peut être déterminée expérimentalement, ou être issue d'un développement analytique préalable, ou encore être fournie dans une base de données, par exemple si elle est fournie par un fabricant de l'implant. La pente obtenue pour une approximation affine de la longueur en fonction du diamètre peut être négative, dans le cas où l'implant, étiré longitudinalement, se rétracte radialement, et si comprimé longitudinalement, il s'élargit radialement. L'étape 500 de correction de position du DMI M du « flow diverter » peut être mise en oeuvre par itérations comme suit.

Des étapes 501 et 502 sont itérées l'une à la suite de l'autre pour chacun des segments Mⱼ dans l'ordre.

A la sous-étape 501, le diamètre du segment Mⱼ est modifié, à partir d'une position courante du segment Mⱼ stockée dans une mémoire d'une base de données accessible de l'unité de traitement. La modification du diamètre Dⱼ du segment Mⱼ est réalisée de façon à loger le segment Mⱼ dans la zone de diamètre minimal de la portion d'artère environnant le segment Mⱼ, de sorte que les parois du segment Mⱼ touchent ladite zone de diamètre minimal. Il est possible de tenir compte d'un diamètre maximal du segment Mⱼ donné par une loi de comportement ; si le diamètre minimal de la portion d'artère est supérieur audit diamètre maximal, le diamètre Dⱼ est alors modifié de sorte à être égal audit diamètre maximal.

Ensuite, à la sous-étape 502, la longueur Lⱼ du segment Mⱼ est corrigée, à l'aide de la loi comportementale du DMI et de la valeur du diamètre corrigé à l'étape 501 pour le segment Mⱼ. Par exemple, si le segment Mⱼ est rétracté radialement lors de l'étape 501, il est allongé à l'étape 502.

Ces étapes 501 et 502 sont répétées pour chacun des segments, pour une itération de l'étape 500.

A une sous-étape 503, la longueur totale L du DMI M est déterminée en faisant la somme des longueurs corrigées Lⱼ des segments M_{j.}

A l'issue de la détermination de la position géométrique, il peut être nécessaire de corriger la position du DMI si l'équilibre mécanique n'est pas suffisamment satisfaisant en partant de la dernière position connue du DMI, soit la position issue de l'étape 400, soit la position issue de l'étape 500. L'unité de traitement peut mettre en oeuvre une simulation de position finale du DMI M selon une seconde variante, où un équilibre mécanique est recherché pour les parois simulées du DMI M. Cette seconde modalité correspond au mode de réalisation 800 pour l'étape de simulation de position finale du DMI M.

On a détaillé des sous-étapes de ce mode de réalisation à la **Figure 4c****.** A la sous-étape 801, une modélisation mécanique du DMI est proposée, basée par exemple sur la méthode des éléments finis qui tient compte des liaisons mécaniques entre les différents segments du DMI, dans sa configuration libre de contraintes mécaniques.

A la sous-étape 802, on transforme, à partir de l'état libre de contrainte lu dans une base de données BD2, les segments Mⱼ dans la dernière position connue. Pour chaque segment Mⱼ, une transformation géométrique est appliquée entre la configuration libre de contraintes mécaniques (position au repos du DMI) et la configuration de départ de la correction mécanique :
- soit issue de l'étape 400 de positionnement du DMI selon la position initiale,
- soit issue d'une étape 500 de simulation géométrique de la position finale du DMI.

A la sous-étape 803, pour chaque segment Mⱼ, une modélisation mécanique du contact avec la surface de l'artère 1 est définie par l'unité de traitement. Enfin, à la sous-étape 804, un calcul non-linéaire de l'équilibre mécanique pour les parois du DMI M est réalisée, une correction de la position des segments Mj du DMI pouvant être appliquée si besoin. La position finale simulée atteinte correspond à une position où les parois du DMI sont à l'équilibre mécanique ainsi calculé.

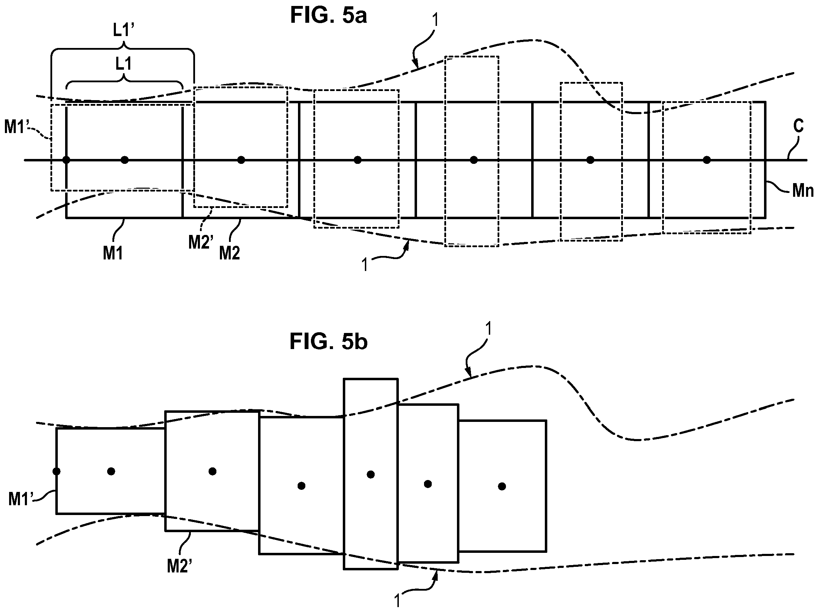

La **Figure 5a** illustre schématiquement sur l'exemple d'une artère 1 la modification de diamètre et de longueur des segments Mⱼ, avec un indice j s'échelonnant de 1 à n = 6, lors d'une première itération des étapes 501 à 503. Sur cette figure, les positions des segments Mⱼ avant correction sont en trait plein, et les positions corrigées M'ⱼ sont en trait pointillé. On constate que les segments de longueur et de diamètre corrigés pour tenir compte des caractéristiques morphologiques de l'artère ne sont plus nécessairement connexes entre eux.

Ensuite, la longueur obtenue à l'étape 503 pour le DMI est comparée à une sous-étape 504 à la longueur obtenue à l'itération précédente des étapes 501 à 503. Si la différence de longueur totale L obtenue est suffisamment proche de zéro (critère de convergence : valeur absolue de la différence entre lesdites longueurs), alors on sort de la boucle d'itération, et les longueurs Lⱼ des segments sont déterminées comme étant les longueurs des segments de l'implant dont on a simulé la position finale. Par exemple, la valeur absolue de la différence est comparée à un seuil prédéterminé. Si, en revanche, la longueur totale L n'est pas déterminée comme suffisamment proche de la longueur totale précédente, alors une nouvelle itération des étapes 501 à 503 est mise en oeuvre. Pour préparer cette nouvelle itération en tenant compte des modifications apportées aux longueurs et diamètres des segments, on ajuste la position des segments de sorte qu'ils soient de nouveau connexes, par exemple à partir du point initial D. Cela est visible sur la **Figure 5b****,** correspondant au même exemple que celui de la Figure 5a pour l'itération suivante des étapes 501 à 503. Le trait plein correspond à la position des segments avant la mise en oeuvre des sous-étapes 501 et 502 pour ladite itération : on constate que les longueurs et diamètres des segments correspondent bien aux positions corrigées de la Figure 5a, et que les segments Mⱼ ont été repositionnés les uns à la suite des autres. Le fait d'avoir repositionné les segments les uns à la suite des autres peut créer de nouveau des non-correspondances entre le diamètre des segments et les parois environnantes de l'artère 1, et donner lieu à un nouveau redimensionnement des segments.

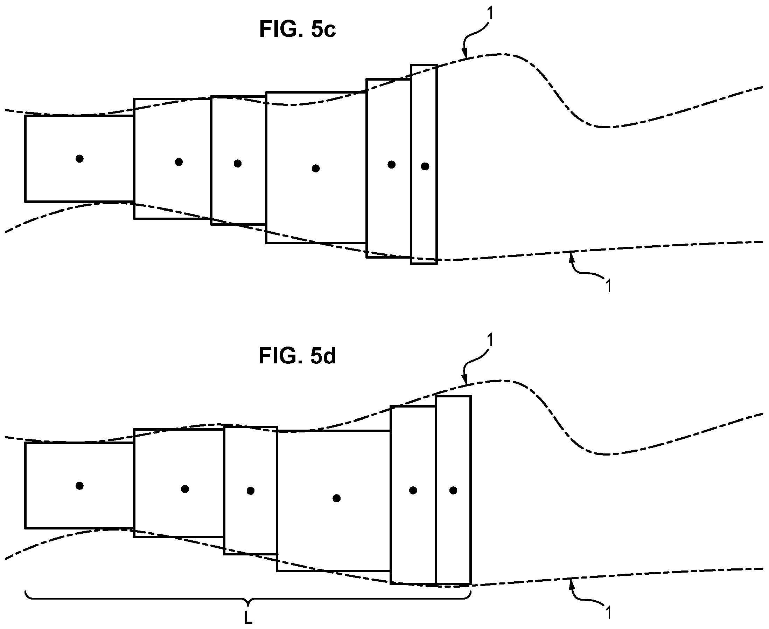

On a représenté à ce titre sur les **Figures 5c et 5d** les deux itérations consécutives à celles des Figures 5a et 5b, pour les mêmes segments. A l'issue de l'itération représentée en Figure 5d, la longueur finale L de l'implant est déterminée comme correspondant à la somme des longueurs des segments.

En sortie de boucle de l'étape 500, les segments sont repositionnés les uns à la suite des autres, et on obtient la position simulée du DMI M après expansion de l'implant. La longueur finale de l'implant une fois déployé est donc prise égale à la somme des longueurs Lⱼ des segments dans cette position simulée.

La méthode de discrétisation décrite ci-avant en relation aux étapes 400 à 500 est applicable à tout type de pathologie de l'artère. Elle permet d'avoir une prédiction de la longueur finale de l'implant après expansion qui soit très proche de la réalité, et ainsi de guider le choix du clinicien. La discrétisation ici proposée est avantageuse, en ce que les longueurs corrigées de chacun des segments du modèle à une itération donnée sont pris en compte pour l'itération suivante.

Selon une variante de réalisation du procédé 40, la correction de position du DMI M à l'étape 500 tient compte non seulement des caractéristiques morphologiques de l'artère, comme le périmètre de l'artère au voisinage d'un segment, mais également du taux de compression longitudinale (en anglais « push/pull ») appliqué par le praticien lors de la pose de l'implant. En effet, si une certaine contrainte est exercée sur l'implant lors de la pose, par exemple dans le sens d'une compression longitudinale générant une extension radiale, l'implant peut adopter localement un périmètre qui dépasse la circonférence du cercle inscrit dans les parois de l'artère. L'implant peut adopter une forme localement non tubulaire (avec des sections radiales non circulaires). Notamment, une contrainte dans le sens longitudinal peut être exercée volontairement lors de la pose de l'implant, pour étendre radialement l'implant et forcer son apposition contre les parois de l'artère, par exemple dans une zone où lesdites parois ne présentent pas une section circulaire.

Par exemple, s'agissant d'un segment de « flow diverter » le diamètre corrigé d'un segment peut être déterminé en fonction du périmètre environnant des parois de l'artère, qui peut tendre à comprimer radialement le segment, mais également en tenant compte de la compression longitudinale exercée lors de la pose qui peut tendre à étendre radialement le segment. Pour la prise en compte du taux de compression longitudinale, le calcul du diamètre corrigé d'un segment Mⱼ à l'étape 501 peut être réalisé en attribuant un poids pour l'influence du périmètre environnant de l'artère.

On comprendra aisément que les autres modalités décrites ci-avant pour la mise en oeuvre de l'étape 500 restent applicables dans ce cas.

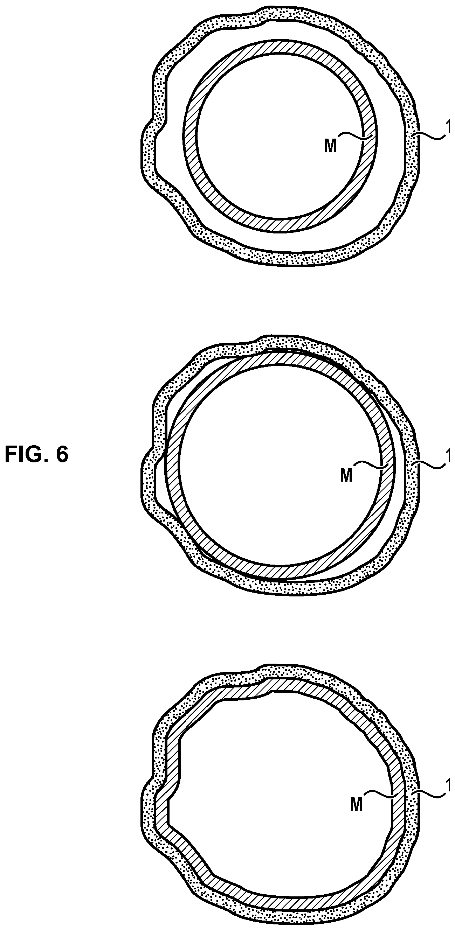

L'impact du taux de compression longitudinale pour la simulation de position après expansion de l'implant est illustré sur les vues schématiques de la **Figure 6****.** Sur une section non circulaire des parois d'une artère 1, on a représenté un DMI M de « flow diverter » ayant une forme cylindrique hors contrainte.

Sur la vue du haut de la Figure 6, on constate que le DMI M sélectionné présente un rayon au repos trop faible pour être apposé correctement contre les parois de l'artère 1.

Pour le même implant visible sur la vue du milieu de la Figure 6, correspondant à une absence de compression longitudinale, le DMI M est étendu radialement, si bien que son diamètre atteint approximativement le diamètre minimal des parois de l'artère 1 sur la section représentée. Toutefois, l'artère n'étant pas localement circulaire, il demeure des zones de l'artère pour lesquelles les parois du modèle d'implant ne sont pas correctement apposées sur les parois de l'artère 1.

Enfin et pour le même implant visible sur la vue du bas de la Figure 6, avec une importante compression longitudinale, l'expansion radiale des parois du DMI M est suffisante pour obtenir une apposition totale de l'implant contre les parois de l'artère, en faisant ici l'hypothèse d'une artère parfaitement rigide. Par ailleurs, la compression longitudinale est suffisante pour imposer à l'implant une forme localement non circulaire après expansion. Dans la réalité, la surface de l'artère n'est pas rigide, et la force radiale appliquée par les parois de l'implant dans le cas d'une compression longitudinale non nulle peut générer des déformations à la fois des parois de l'implant et de la surface de l'artère.

L'application d'un « push/pull » lors de la pose de l'implant permet donc, pour un implant de type « flow diverter » d'obtenir une bonne apposition de l'implant contre les parois de l'artère, notamment sur des zones où l'artère est localement non circulaire. La variante de l'étape 500 décrite ci-avant permet de tenir compte de la possibilité d'appliquer un « push/pull », et permet donc de fiabiliser la simulation de l'implant.

À l'issue de la détermination de la position corrigée du DMI M correspondant à la position simulée de l'implant, une étape 600 supplémentaire de détermination du degré d'apposition de l'implant est mise en oeuvre, comme représenté en Figure 4a.

Une fois la position du DMI M d'implant après expansion simulée, l'unité de traitement peut calculer le degré d'apposition comme étant la proportion de la surface de l'implant sur laquelle les parois de l'implant présentent une bonne apposition contre les parois de l'artère. Par exemple, pour les cas exposés précédemment en relation à la Figure 6, la vue du haut correspond à un degré d'apposition de 0% et la vue du bas correspond à un degré d'apposition de 100%. On comprendra aisément que, dans le cas d'une pathologie de type sac anévrismal, et pour un implant de type « flow diverter », il n'est pas souhaité d'obtenir une bonne apposition des parois de l'implant contre les parois du sac anévrismal ; en revanche, il est souhaitable d'obtenir une bonne apposition du dispositif contre les autres zones de l'artère.

Dans le cas d'un DMI qui ne serait pas un « flow diverter », comme par exemple une cage intra-anévrismale, l'apposition pourrait être déterminée en considérant la force mécanique de contact imposée par la paroi de l'artère sur les parois de l'implant, ou encore, en considérant les distances entre des noeuds des parois du DMI et les parois avoisinantes d'artère (le DMI étant considéré comme correctement apposé en-dessous d'un certain seuil de distance).

De manière optionnelle, le procédé 40 peut comprendre une étape supplémentaire 700 de détermination de la porosité des parois du DMI M. Cette étape pourrait alternativement être mise en oeuvre avant l'étape 600 de détermination de degré d'apposition des parois du DMI. La détermination de porosité des parois du DMI M est faite selon tout procédé connu de l'état de la technique.

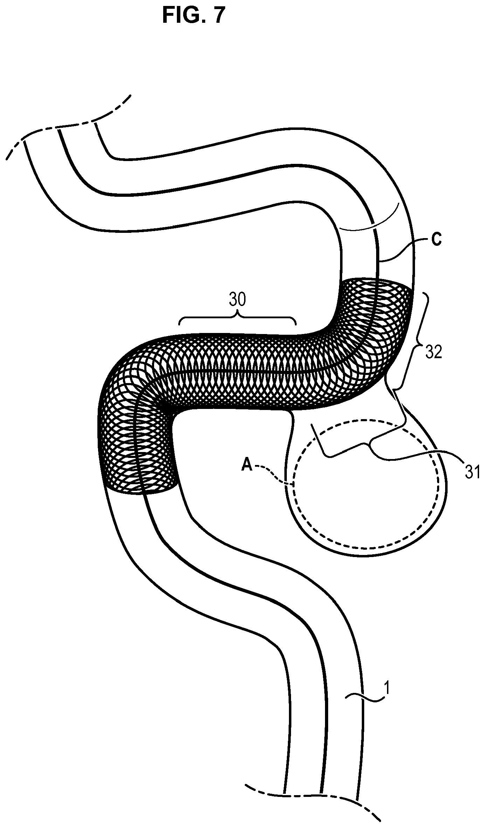

A l'issue de la détermination du degré d'apposition pour le DMI M, indépendamment de la mise en oeuvre d'une étape 700 de détermination de porosité des parois du DMI, l'unité de traitement peut afficher, via le dispositif d'affichage 21, une vue tridimensionnelle de l'artère traitée avec la position tridimensionnelle simulée de l'implant, et afficher en outre une cartographie du degré d'apposition sur toute la surface de l'implant. Une telle vue est représentée de manière schématique en **Figure 7****,** où est visible une artère 1 traitée avec un « flow diverter » au niveau d'une zone A le long d'une ligne centrale C. Pour les zones 30 et 32, l'apposition de l'implant contre les parois de l'artère 1 est plus importante qu'au niveau du collet de l'anévrisme, sur la zone 31.

A l'issue d'une itération du procédé 40 de détermination de positionnement en position déployée du DMI, si la position finale déterminée est insuffisamment proche des parois de l'artère (par exemple, si l'apposition finale est jugée insuffisante), on peut prévoir un nouveau modèle de DMI de sorte à mieux approcher les parois de l'artère. Il est possible de tenir compte d'un diamètre maximal des segments Mⱼ donné par une loi comportementale ; si le diamètre minimal de la portion de l'artère est supérieur audit diamètre maximal, le diamètre Dⱼ est alors modifié de sorte à être égal audit diamètre maximal.

En alternative, les étapes 400 et 500 telles que décrites ci-avant peuvent être réitérées avec une référence d'implant différente, ayant par exemple un diamètre nominal différent. La comparaison de plusieurs positions finales de DMI obtenues pour différentes références d'implant, en faisant éventuellement varier le positionnement du point distal D et/ou du point proximal P, permet au clinicien de sélectionner la référence d'implant et les modalités d'intervention les plus adaptées au traitement de la pathologie que présente l'artère.

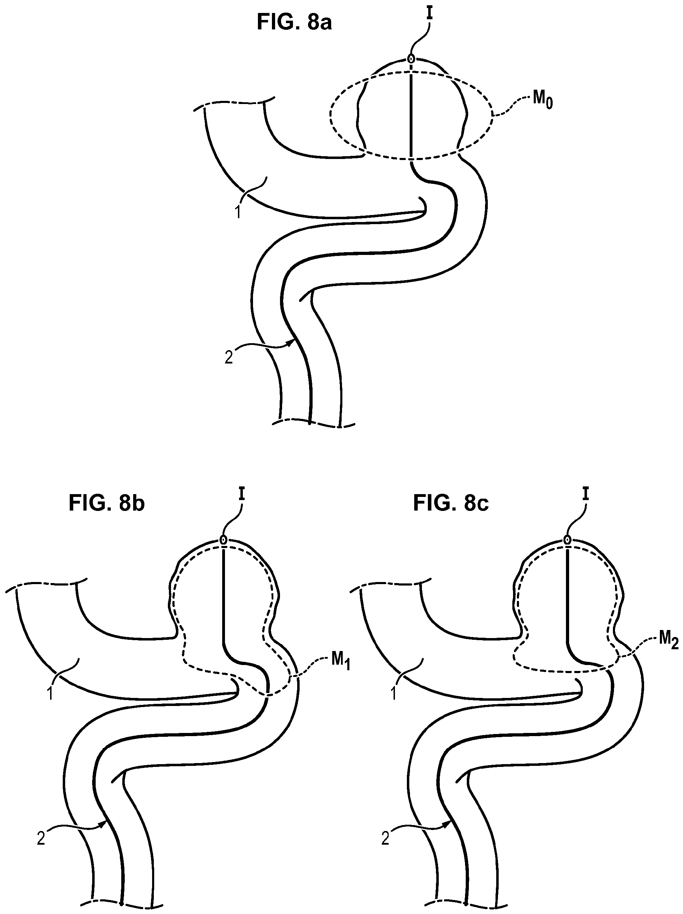

La **Figure 8a** illustre le résultat de l'étape de positionnement du DMI dans sa position initiale, pour un DMI de type cage intra-anévrismale. A l'issue de l'étape 400, on a positionné dans l'image tridimensionnelle de l'artère 1 un implant de ce type, selon une position initiale M₀, le long de la ligne centrale 2 calculée par exemple selon l'étape 300 décrite précédemment.

La **Figure 8b** représente le résultat visible sur l'interface graphique à l'issue d'une itération de l'étape 500 de simulation de position finale du DMI, en tenant compte uniquement des contraintes géométriques exercées par les parois intra-sacculaires. On obtient ainsi une position M₁ du DMI.

Enfin, sur la **Figure** 8c, on a représenté le résultat visible sur l'interface graphique, à l'issue d'une itération de l'étape 800 de simulation de position finale du DMI tenant compte des contraintes mécaniques exercées par les parois, en cherchant à résoudre l'équilibre mécanique pour les parois du DMI selon la position M₁ précédemment déterminée.

Pour cet exemple, beaucoup plus de temps de calcul aurait été requis pour calculer l'équilibre mécanique selon l'étape 800 à partir de la position initiale M₀, comparativement au temps de calcul requis ici pour calculer la position M₂ à partir de la position M₁ pour laquelle on a déjà tenu compte de contraintes géométriques exercées par les parois intra-sacculaires.

## Revendications

1. Procédé de détermination du positionnement en position déployée d'un dispositif médical implantable, ou DMI, expansible, ledit dispositif médical étant destiné à être déployé dans une structure vasculaire (1), le procédé comprenant, à partir d'une image tridimensionnelle d'une région d'intérêt comprenant la structure vasculaire dans laquelle un point de positionnement (D) du DMI a été défini, les étapes suivantes, mises en oeuvre par ordinateur :
- détermination (300) d'une ligne centrale (C) de l'artère, la détermination (300) de la ligne centrale (C) consistant à placer des points à des positions longitudinales différentes le long de la structure vasculaire, de sorte à minimiser un temps de trajet de fluide le long desdits points (Cᵢ) entre un point d'entrée (I) dans la structure vasculaire et un point de sortie (S) de la structure vasculaire, lesdits points formant la ligne centrale (C) ;
- positionnement (400) du DMI (M) selon une position initiale, autour de la ligne centrale (C), le DMI étant déployé le long de la ligne centrale (C) et centré sur cette ligne centrale (C) ;
- simulation (500, 800) de la position finale (M) du DMI après déploiement, en fonction de contraintes exercées par les parois de la structure vasculaire (1) sur le DMI, la simulation de la position finale du DMI étant réalisée en fonction d'un taux de compression longitudinale destiné à être appliqué au DMI au cours de son implantation ;
- détermination (600) d'un degré d'apposition du DMI sur les parois de la structure vasculaire, en position finale, de manière à estimer l'apposition de l'implant contre les parois de la structure vasculaire, le degré d'apposition étant la proportion de la surface du DMI sur laquelle les parois de l'implant présentent une bonne apposition contre les parois de la structure vasculaire ;
la position finale et le degré d'apposition permettant de choisir un DMI adapté à la structure vasculaire (1).

2. Procédé selon la revendication 1, dans lequel le temps de trajet entre les points (Ci) placés est minimisé en utilisant un algorithme de descente de gradient, lesdits points (Ci) formant la ligne centrale (C).

3. Procédé selon l'une des revendications 1 ou 2, dans lequel la détermination (300) de la ligne centrale (C) comprend des sous-étapes consistant à :
- tracer (302) la ligne centrale (C) passant par lesdits points (Cᵢ) ;
- discrétiser (303) la structure vasculaire de sorte à obtenir une succession de portions tridimensionnelles de la structure vasculaire le long de la ligne centrale.

4. Procédé selon l'une des revendications 1 à 3, dans lequel l'étape de positionnement du DMI selon la position initiale est suivie d'une étape de discrétisation du DMI en un ensemble de segments tridimensionnels longitudinaux,
l'étape de simulation de la position finale (500) prenant en compte des contraintes géométriques exercées par les parois de la structure vasculaire sur le DMI, par les sous-étapes suivantes :
a) pour chaque segment (Mⱼ), modification (501) du diamètre dudit segment en fonction du périmètre de la structure vasculaire au voisinage dudit segment, et modification (502) de la longueur dudit segment, à l'aide d'une loi comportementale du DMI reliant ladite longueur audit diamètre du segment,
b) calcul (503) d'une longueur du DMI comme étant la somme des longueurs des segments à l'issue de l'étape a),
c) comparaison (504) des deux dernières longueurs calculées à l'étape b),
- si la valeur absolue de la différence entre les deux dernières longueurs de DMI calculées est inférieure à un seuil, la longueur du DMI en position finale étant la dernière longueur calculée à l'étape b) et
- si ladite valeur absolue de la différence est supérieure audit seuil, l'étape de correction comprenant une étape d'ajustement de la position de chaque segment du DMI en tenant compte des modifications de longueur des segments à l'issue de l'étape a), et une répétition des étapes a) à c) avec les segments ainsi modifiés.

5. Procédé selon l'une des revendications 1 à 4, dans lequel l'étape de simulation de la position finale (800) prend en compte des contraintes mécaniques exercées par les parois de la structure vasculaire sur le DMI, par les sous-étapes suivantes :
- affectation (801) d'une modélisation mécanique à chaque segment (Mⱼ),
- pour chaque segment (Mⱼ), transformation (802) du diamètre (Dⱼ) dudit segment, et affectation d'un contact mécanique entre ledit segment (Mⱼ) et la structure vasculaire (1),
- résolution (803) de l'équilibre mécanique du DMI pour obtenir une position finale du DMI.

6. Procédé selon l'une des revendications 1 à 5, comprenant une étape préliminaire (250) de vérification de la reconstruction 3D de la surface de la structure vasculaire dans la région d'intérêt, ladite vérification étant réalisée par seuillage.

7. Procédé selon l'une des revendications 1 à 6, comprenant après l'étape de détermination (300) de la ligne centrale une étape de correction d'un ou plusieurs abouchements non physiologiques de ladite surface, tels qu'une fusion artère-artère ou une fusion artère-sac anévrismal.

8. Procédé selon la revendication 4, dans lequel, si la position finale déterminée pour le DMI est insuffisamment proche des parois de la structure vasculaire, l'étape de simulation (500) de position finale est réitérée avec des segments (Mⱼ) de diamètres plus élevés de sorte à rapprocher les segments des parois de la structure vasculaire.

9. Procédé selon la revendication 6, comprenant une étape supplémentaire de représentation graphique tridimensionnelle de la structure vasculaire dans la région d'intérêt, comprenant la ligne centrale (C) déterminée, ainsi que le degré d'apposition.

10. Procédé selon la revendication 3, dans lequel la loi comportementale permettant de caractériser la contraction d'un segment du DMI est une relation fonction des paramètres géométriques et mécaniques du DMI.

11. Procédé selon l'une des revendications 1 à 10 dans lequel le DMI est de type « flow diverter », ou cage intra-anévrismale, ou « stent laser-cut ».

12. Procédé selon l'une des revendications 1 à 11, comprenant une étape (700) de détermination d'une porosité des parois du DMI en position finale.

13. Produit programme d'ordinateur comprenant des instructions de code pour l'exécution du procédé selon l'une des revendications 1 à 12, lorsqu'il est exécuté par des moyens de traitement d'une unité de traitement.

14. Unité de traitement (20) configurée pour obtenir une image tridimensionnelle de structure vasculaire acquise d'une unité d'acquisition (22), et configurée en outre pour simuler une position finale d'un DMI expansible, par la mise en oeuvre du procédé selon l'une des revendications 1 à 12.

## Patentansprüche

1. Verfahren zum Bestimmen der Positionierung eines expandierbaren implantierbaren Medizinprodukts, oder IMD, in entfalteter Position, wobei das Medizinprodukt dafür bestimmt ist, in einer Gefäßstruktur (1) entfaltet zu werden, wobei das Verfahren, ausgehend von einem die Gefäßstruktur umfassenden dreidimensionalen Bild einer Region von Interesse, in dem ein Positionierungspunkt (D) des IMD definiert wurde, die folgenden, mittels Computer implementierten Schritte umfasst:
- Bestimmen (300) einer Mittellinie (C) der Arterie, wobei das Bestimmen (300) der Mittellinie (C) darin besteht, Punkte an verschiedenen Längspositionen entlang der Gefäßstruktur so zu setzen, dass eine Fließzeit von Fluid entlang der Punkte (Cᵢ) zwischen einem Eintrittspunkt (I) in die Gefäßstruktur und einem Austrittspunkt (S) aus der Gefäßstruktur minimiert wird, wobei die Punkte die Mittellinie (C) bilden;
- Positionieren (400) des IMD (M) in einer Anfangsposition um die Mittellinie (C) herum, wobei das IMD entlang der Mittellinie (C) entfaltet und auf dieser Mittellinie (C) zentriert wird;
- Simulieren (500, 800) der Endposition (M) des IMD nach Entfalten abhängig von Einschränkungen, die von den Wänden der Gefäßstruktur (1) auf das IMD ausgeübt werden, wobei das Simulieren der Endposition des IMD abhängig von einem Längskompressionsgrad, der während seiner Implantation an das IMD angelegt werden soll, ausgeführt wird;
- Bestimmen (600) eines Grades der Anlagerung des IMD in der Endposition an den Wänden der Gefäßstruktur, um die Anlagerung des Implantats an den Wänden der Gefäßstruktur abzuschätzen, wobei es sich beim Grad der Anlagerung um den Anteil der Oberfläche des IMD handelt, auf dem die Wände des Implantats eine gute Anlagerung an den Wänden der Gefäßstruktur aufweisen;
wobei es die Endposition und der Grad der Anlagerung ermöglichen, ein für die Gefäßstruktur (1) geeignetes IMD auszuwählen.

2. Verfahren nach Anspruch 1, wobei die Fließzeit zwischen den gesetzten Punkten (Ci) minimiert wird, indem ein Gradientenabstiegsalgorithmus verwendet wird, wobei die Punkte (Ci) die Mittellinie (C) bilden.

3. Verfahren nach einem der Ansprüche 1 oder 2, wobei das Bestimmen (300) der Mittellinie (C) Teilschritte umfasst, die darin bestehen:
- die durch die Punkte (Cᵢ) verlaufende Mittellinie (C) einzuzeichnen (302);
- die Gefäßstruktur so zu diskretisieren (303), dass man eine Serie von dreidimensionalen Abschnitten der Gefäßstruktur entlang der Mittellinie erhält.

4. Verfahren nach einem der Ansprüche 1 bis 3, wobei dem Schritt des Positionierens des IMD in der Anfangsposition ein Schritt des Diskretisierens des IMD in eine Menge von dreidimensionalen Längssegmenten folgt,
wobei der Schritt des Simulierens der Endposition (500) geometrische Einschränkungen, die von den Wänden der Gefäßstruktur auf das IMD ausgeübt werden, mittels der folgenden Teilschritte berücksichtigt:
a) Modifizieren (501), bei jedem Segment (Mⱼ), des Durchmessers des Segments abhängig vom Umfang der Gefäßstruktur in der Nähe des Segments, und Modifizieren (502) der Länge des Segments mithilfe eines Verhaltensgesetzes des IMD, welches die Länge mit dem Durchmesser des Segments in Beziehung setzt,
b) Berechnen (503) einer Länge des IMD als Summe der Längen der Segmente am Ende von Schritt a),
c) Vergleichen (504) der zwei letzten in Schritt b) berechneten Längen,
- wobei, wenn der Absolutwert der Differenz zwischen den zwei letzten berechneten Längen des IMD kleiner als eine Schwelle ist, die Länge des IMD in der Endposition die letzte in Schritt b) berechnete Länge ist, und
- wobei, wenn der Absolutwert der Differenz größer als die Schwelle ist, der Korrekturschritt einen Schritt des Anpassens der Position jedes Segments des IMD unter Berücksichtigung der Modifizierungen der Länge der Segmente am Ende von Schritt a), und ein Wiederholen der Schritte a) bis c) mit den so modifizierten Segmenten umfasst.

5. Verfahren nach einem der Ansprüche 1 bis 4, wobei der Schritt des Simulierens der Endposition (800) mechanische Einschränkungen, die von den Wänden der Gefäßstruktur auf das IMD ausgeübt werden, mittels der folgenden Teilschritte berücksichtigt:
- Zuordnen (801) einer mechanischen Modellierung zu jedem Segment (Mⱼ),
- Transformieren (802), bei jedem Segment (Mⱼ), des Durchmessers (Dⱼ) des Segments, und Zuordnen eines mechanischen Kontakts zwischen dem Segment (Mⱼ) und der Gefäßstruktur (1),
- Lösen (803) des mechanischen Gleichgewichts des IMD, um eine Endposition des IMD zu erhalten.

6. Verfahren nach einem der Ansprüche 1 bis 5, das einen vorausgehenden Schritt (250) des Überprüfens der 3D-Rekonstruktion der Oberfläche der Gefäßstruktur in der Region von Interesse umfasst, wobei die Überprüfung mittels Schwellenwertbestimmung ausgeführt wird.

7. Verfahren nach einem der Ansprüche 1 bis 6, das nach dem Schritt des Bestimmens (300) der Mittellinie einen Schritt des Korrigierens einer oder mehrerer nicht-physiologischer Ausmündungen der Oberfläche, wie etwa einer Verbindung Arterie - Arterie oder einer Verbindung Arterie - Aneurysmasack umfasst.

8. Verfahren nach Anspruch 4, wobei, wenn die für das IMD bestimmte Endposition nicht ausreichend nahe an den Wänden der Gefäßstruktur liegt, der Schritt des Simulierens (500) der Endposition mit Segmenten (Mⱼ) mit größeren Durchmessern wiederholt wird, um die Segmente den Wänden der Gefäßstruktur anzunähern.

9. Verfahren nach Anspruch 6, das einen zusätzlichen Schritt der dreidimensionalen grafischen Darstellung der Gefäßstruktur in der Region von Interesse umfasst, welche die bestimmte Mittellinie (C) sowie den Grad der Anlagerung umfasst.

10. Verfahren nach Anspruch 3, wobei es sich bei dem Verhaltensgesetz, das es ermöglicht, die Kontraktion eines Segments des IMD zu charakterisieren, um eine Beziehung handelt, die von den geometrischen und mechanischen Parametern des IMD abhängig ist.

11. Verfahren nach einem der Ansprüche 1 bis 10, wobei das IMD vom Typ "Flow-Diverter" oder Intra-Aneurysma-Cage, oder "Laser-cut Stent" ist.

12. Verfahren nach einem der Ansprüche 1 bis 11, das einen Schritt (700) des Bestimmens einer Porosität der Wände des IMD in der Endposition umfasst.

13. Computerprogrammprodukt, das Codeanweisungen umfasst zum Ausführen des Verfahrens nach einem der Ansprüche 1 bis 12, wenn es von Verarbeitungsmitteln einer Verarbeitungseinheit ausgeführt wird.

14. Verarbeitungseinheit (20), die dafür ausgelegt ist, ein dreidimensionales Bild einer Gefäßstruktur, das mit einer Aufzeichnungseinheit (22) aufgezeichnet wurde, zu erhalten, und weiter dafür ausgelegt ist, eine Endposition eines expandierbaren IMD mittels Implementieren des Verfahrens nach einem der Ansprüche 1 bis 12 zu simulieren.

## Claims

1. Method for determining the positioning in deployed position of an expandable implantable medical device, or IMD, said medical device being intended to be deployed in a vascular structure (1), the method comprising, from a three-dimensional image of a region of interest comprising the vascular structure in which a positioning point (D) of the IMD has been defined, the following steps implemented by computer:
- determination (300) of a centreline (C) of the artery, the determination (300) of the centreline (C) consisting in placing points at different longitudinal positions along the vascular structure, so as to minimise a travel time of fluid along said points (Cᵢ) between an input point (I) in the vascular structure and an output point (S) in the vascular structure, said points forming the centreline (C);
- positioning (400) of the IMD (M) according to an initial position, around the centreline (C), the IMD being deployed along the centreline (C) and centred on said centreline (C);
- simulation (500, 800) of the final position (M) of the IMD after deployment, as a function of the stresses exerted by the walls of the vascular structure (1) on the IMD, the simulation of the final position of the IMD is carried out as a function of a level of longitudinal push-pull intended to be applied to the IMD during its implantation;
- determination (600) of a degree of apposition of the IMD on the walls of the vascular structure, in final position, to estimate the apposition of the implant against the walls of the vascular structure, the degree of apposition being the proportion of the surface of the IMD on which the walls of the implant have good apposition against the walls of the vascular structure.
the final position and the degree of apposition allowing an IMD to be chosen adapted to the vascular structure.

2. Method according to claim 1, wherein the travel time between the points (Cᵢ) placed is minimised using a gradient descent algorithm, said points (Cᵢ) forming the centreline (C).

3. Method according to one of claims 1 or 2, wherein the determining (300) of the centreline (C) comprises the sub-steps consisting in:
- plotting (302) the centreline (C) passing through said points (Cᵢ);
- discretising (303) the vascular structure so as to obtain a succession of three-dimensional portions of the vascular structure along the centreline.

4. Method according to one of claims 1 to 3, wherein the step of positioning the IMD according to the initial position is followed by a step of discretisation of the IMD into a set of longitudinal three-dimensional segments,
the step of simulation of the final position (500) taking into account the geometric stresses exerted by the walls of the vascular structure on the IMD, by the following sub-steps:
a) for each segment (Mⱼ), modification (501) of the diameter of said segment as a function of the perimeter of the vascular structure in the vicinity of said segment, and modification (502) of the length of said segment, by means of a behavioural law of the IMD linking said length to said diameter of the segment,
b) calculation (503) of a length of the IMD as being the sum of the lengths of the segments at the end of step a),
c) comparison (504) of the two final lengths calculated at step b),
- if the absolute value of the difference between the two calculated final lengths of IMD is below a threshold, the length of the IMD in final position is the final length calculated at step b) and
- if said absolute value of the difference is above said threshold, the correction step comprises a step of adjustment of the position of each segment of the IMD while taking account of the modifications of length of the segments at the end of step a), and a repetition of steps a) to c) with the segments thus modified.

5. Method according to one of claims 1 to 4, wherein the step of simulation of the final position (800) takes into account the mechanical stresses exerted by the walls of the vascular structure on the IMD, by the following sub-steps:
- allocation (801) of a mechanical modelling to each segment (Mⱼ),
- for each segment (Mⱼ), transformation (802) of the diameter (Dⱼ) of said segment, and allocation of a mechanical contact between said segment (Mⱼ) and the vascular structure (1),
- resolution (803) of the mechanical equilibrium of the IMD to obtain a final position of the IMD.

6. Method according to one of claims 1 to 5, comprising a preliminary step (250) of verification of the 3D reconstruction of the surface of the vascular structure in the region of interest, said verification being carried out by thresholding.

7. Method according to one of claims 1 to 6, comprising after the step of determination (300) of the centreline a step of correction of one or more non-physiological anastomoses of said surface, such as an artery-artery fusion or an artery-aneurysmal sac fusion.

8. Method according to claim 4, wherein, if the final position determined for the IMD is insufficiently close to the walls of the vascular structure, the step of simulation (500) of the final position is reiterated with segments (Mⱼ) of larger diameters, so as to bring closer together the segments of the walls of the vascular structure.

9. Method according to one of claims 4 or 8, wherein the behavioural law making it possible to characterise the contraction of a segment of the IMD is a relationship dependent on the geometric and mechanical parameters of the IMD.

10. Method according to claim 6, comprising an additional step of three-dimensional graphic representation of the vascular structure in the region of interest, comprising the determined centreline (C), as well as the degree of apposition.

11. Method according to one of claims 1 to 10 wherein the IMD is of flow diverter, or intra-aneurysmal cage, or stent laser-cut type.

12. Method according to one of claims 1 to 11, comprising a step (700) of determination of a porosity of the walls of the IMD in final position.

13. Computer programme product comprising code instructions for the execution of the method according to one of claims 1 to 12, when it is executed by processing means of a processing unit.

14. Processing unit (20) configured to obtain a three-dimensional vascular structure image acquired from an acquisition unit (22), and further configured to simulate a final position of an expandable IMD, by the implementation of the method according to one of claims 1 to 12.
